# EUROPEAN PATENT SPECIFICATION

(11) **EP 2 635 222 B1**
(45) Date of publication and mention of the grant of the patent: **09.09.2020**
(21) Application number: 11784882.0
(22) Date of filing: 04.11.2011
(51) Int. Cl.: A61B 18/14, A61B 17/32, A61B 17/00, A61B 18/00, A61B 17/064

(54) **SURGICAL INSTRUMENT WITH MODULAR END EFFECTOR AND DETECTION FEATURE**
CHIRURGISCHES INSTRUMENT MIT MODULAREM ENDEFFEKTOR UND DETEKTOR
INSTRUMENT CHIRURGICAL AVEC EFFECTEUR D'EXTRÉMITÉ MODULAIRE ET FONCTIONNALITÉ DE DÉTECTION

(30) Priority: 19.05.2011 US 201161487846 P; 05.11.2010 US 410603 P; 18.10.2011 US 201113275563
(43) Date of publication of application: 11.09.2013
(73) Proprietor: Ethicon LLC, 00969 Guaynabo (PR)
(72) Inventor: HOUSER, Kevin, L., Springboro OH 45066 (US); MONSON, Gavin, M., Oxford OH 45056 (US)
(74) Representative: Tunstall, Christopher Stephen
(86) International application number: PCT/US2011/059354
(87) International publication number: WO 2012/061720

(56) References cited:
- EP-A1- 0 947 167
- EP-A1- 2 027 819
- JP-A- H03 126 447
- JP-A- 2004 290 516
- US-A1- 2003 093 103
- US-A1- 2004 133 189
- US-A1- 2009 275 940
- US-A1- 2010 114 090

## Description

### BACKGROUND

In some settings, endoscopic surgical instruments may be preferred over traditional open surgical devices since a smaller incision may reduce the post-operative recovery time and complications. Consequently, some endoscopic surgical instruments may be suitable for placement of a distal end effector at a desired surgical site through a cannula of a trocar. These distal end effectors may engage tissue in a number of ways to achieve a diagnostic or therapeutic effect (e.g., endocutter, grasper, cutter, stapler, clip applier, access device, drug/gene therapy delivery device, and energy delivery device using ultrasound, RF, laser, etc.). Endoscopic surgical instruments may include a shaft between the end effector and a handle portion, which is manipulated by the clinician. Such a shaft may enable insertion to a desired depth and rotation about the longitudinal axis of the shaft, thereby facilitating positioning of the end effector within the patient.

US 2004/133189 A1 discloses a surgical operation apparatus including a surgical instrument; a drive device that generates an energy with which the surgical instrument works; an energy transmission cable; a first connector provided on one end of the cable; an energy release unit that releases the energy out of the first connector; a second connector that is detachable from the first connector; an energy receiving unit that receives the energy released from the energy release unit, and an operation functioning unit that functions based on the energy received. US 2009/275940 A1 discloses a surgical tool system with a powered tool that includes a memory in which data describing the characteristics of the power signals that are to be sourced to the tool are stored. A control console based on the data in the tool memory sources the power signal to the tool. Both the data signals written out from the tool memory and the power signals from the console are exchanged over common console terminals and common tool terminals. An isolation circuit in the tool prevents the applied power signals from the console from adversely affecting the tool memory. JPH03126447 refer to a further patent document relevant for the present invention.

Examples of endoscopic surgical instruments include those disclosed in U.S. Pat. Pub. No. 2006/0079874, entitled "Tissue Pad Use with an Ultrasonic Surgical Instrument," published April 13, 2006, U.S. Pat. Pub. No. 2007/0191713, entitled "Ultrasonic Device for Cutting and Coagulating," published August 16, 2007, U.S. Pat. Pub. No. 2007/0282333, entitled "Ultrasonic Waveguide and Blade," published December 6, 2007, U.S. Pat. Pub. No. 2008/0200940, entitled "Ultrasonic Device for Cutting and Coagulating," published August 21, 2008, U.S. Pat. Pub. No. 2011/0015660, entitled "Rotating Transducer Mount for Ultrasonic Surgical Instruments," published January 20, 2011, Pat. No. 6,500,176, entitled "Electrosurgical Systems and Techniques for Sealing Tissue," issued December 31, 2002, U.S. Pat. Pub. No. 2011/0087218, entitled "Surgical Instrument Comprising First and Second Drive Systems Actuatable by a Common Trigger Mechanism," published April 14, 2011. Additionally, such surgical tools may include a cordless transducer such as that disclosed in U.S. Pat. Pub. No. 2009/0143797, entitled "Cordless Hand-held Ultrasonic Cautery Cutting Device," published June 4, 2009, In addition, the surgical instruments may be used, or adapted for use, in robotic-assisted surgery settings such as that disclosed in U.S. Pat. No. 6,783,524, entitled "Robotic Surgical Tool with Ultrasound Cauterizing and Cutting Instrument," issued August 31, 2004,

While several systems and methods have been made and used for surgical instruments, it is believed that no one prior to the inventors has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1 depicts a schematic view of an exemplary medical device having an internal power source;
FIG. 2 depicts a perspective view of an exemplary medical device having an internal power source;
FIG. 3 depicts a partial perspective view of an exemplary transmission assembly;
FIG. 4 depicts a partial perspective view of an exemplary transmission assembly for delivering ultrasonic energy;
FIG. 5 depicts a partial perspective view of an exemplary transmission assembly for delivering RF energy;
FIG. 6 depicts a partial perspective view of an exemplary transmission assembly for delivering staples;
FIG. 7 depicts a side cross sectional view of an exemplary handle assembly;
FIG. 8 depicts a side perspective, partially cross sectional view of a handle assembly and two transmission assemblies, according to an embodiment of the present invention;
FIG. 9 depicts a side cross sectional view of a handle assembly for use with bipolar and monopolar RF transmission assemblies;
FIG. 10 depicts a side cross sectional view of a partial handle assembly with a transmission assembly with a identification device;
FIG. 11 depicts a schematic view of an exemplary amplifier, according to an embodiment of the present invention; and
FIG. 12 depicts a schematic view of an exemplary reconstruction filter.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is defined by independent claim 1.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the scope of independent claim 1. Accordingly, the drawings and descriptions should be regarded as illustrative in nature and not restrictive.

It will be appreciated that the terms "proximal" and "distal" are used herein with reference to a clinician gripping a handpiece assembly. Thus, an end effector is distal with respect to the more proximal handpiece assembly. It will be further appreciated that, for convenience and clarity, spatial terms such as "top" and "bottom" also are used herein with respect to the clinician gripping the handpiece assembly. However, surgical instruments are used in many orientations and positions, and these terms are not intended to be limiting and absolute.

### I. Medical Devices for Use With Insertable or Reclaimable Components

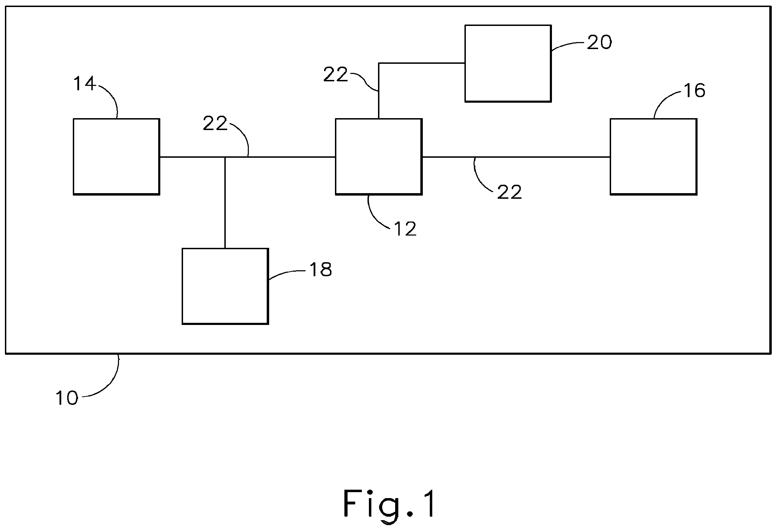

FIG. 1 shows components of an exemplary medical device (10) in diagrammatic block form. As shown, medical device (10) comprises a control module (12), a power source (14), and an end effector (16). Merely exemplary power sources (14) may include NiMH batteries, Li-ion batteries (e.g., prismatic cell type lithium ion batteries, etc.), Ni-Cad batteries, or any other type of power source as may be apparent to one of ordinary skill in the art in light of the teachings herein. Control module (12) may comprise a microprocessor, an application specific integrated circuit (ASIC), memory, a printed circuit board (PCB), a storage device (such as a solid state drive or hard disk), firmware, software, or any other suitable control module components as will be apparent to one of ordinary skill in the art in light of the teachings herein. Control module (12) and power source (14) are coupled by an electrical connection (22), such as a cable and/or traces in a circuit board, etc., to transfer power from power source (14) to control module (12). Alternatively, power source (14) may be selectively coupled to control module (12). This allows power source (14) to be detached and removed from medical device (10), which may further allow power source (14) to be readily recharged or reclaimed for resterilization and reuse, such as in accordance with the various teachings herein. In addition or in the alternative, control module (12) may be removed for servicing, testing, replacement, or any other purpose as will be apparent to one of ordinary skill in the art in view of the teachings herein.

End effector (16) is coupled to control module (12) by another electrical connection (22). End effector (16) is configured to perform a desired function of medical device (10). By way of example only, such function may include cauterizing tissue, ablating tissue, severing tissue, ultrasonically vibrating, stapling tissue, or any other desired task for medical device (10). End effector (16) may thus include an active feature such as an ultrasonic blade, a pair of clamping jaws, a sharp knife, a staple driving assembly, a monopolar RF electrode, a pair of bipolar RF electrodes, a thermal heating element, and/or various other components. End effector (16) may also be removable from medical device (10) for servicing, testing, replacement, or any other purpose as will be apparent to one of ordinary skill in the art in view of the teachings herein. In some versions, end effector (16) is modular such that medical device (10) may be used with different kinds of end effectors Various other configurations of end effector (16) may be provided for a variety of different functions depending upon the purpose of medical device (10) as will be apparent to those of ordinary skill in the art in view of the teachings herein. Similarly, other types of components of a medical device (10) that may receive power from power source (14) will be apparent to those of ordinary skill in the art in view of the teachings herein.

Medical device (10) of the present example includes a trigger (18) and a sensor (20), though it should be understood that such components are merely optional. Trigger (18) is coupled to control module (12) and power source (14) by electrical connection (22). Trigger (18) may be configured to selectively provide power from power source (14) to end effector (16) (and/or to some other component of medical device (10)) to activate medical device (10) when performing a procedure. Sensor (20) is also coupled to control module (12) by an electrical connection (22) and may be configured to provide a variety of information to control module (12) during a procedure. By way of example only, such configurations may include sensing a temperature at end effector (16) or determining the oscillation rate of end effector (16). Data from sensor (20) may be processed by control module (12) to effect the delivery of power to end effector (16) (e.g., in a feedback loop, etc.). Various other configurations of sensor (20) may be provided depending upon the purpose of medical device (10) as will be apparent to those of ordinary skill in the art in view of the teachings herein. Of course, as with other components described herein, medical device (10) may have more than one sensor (20), or sensor (20) may simply be omitted if desired.

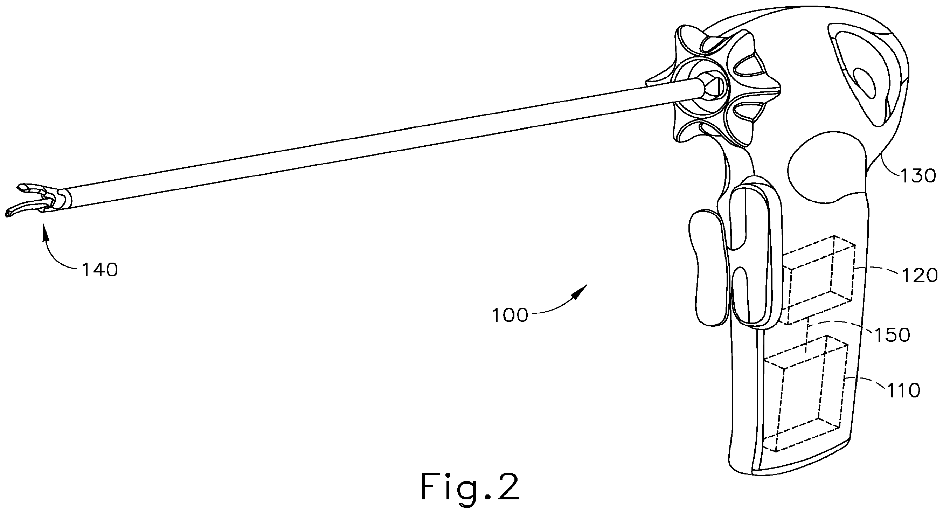

FIG. 2 depicts a merely exemplary form that medical device (10) may take. In particular, FIG. 2 shows a medical device (100) comprising a power source (110), a control module (120), a housing (130), end effector (140), and an electrical connection (150). In the present example, power source (110) is located internally within housing (130) of medical device (100). Alternatively, power source (110) may only partially extend into housing (130) and may be selectively attachable to a portion of housing (130). In yet a further exemplary configuration, a portion of housing (130) may extend into power source (110) and power source (110) may be selectively attachable to the portion of housing (130). Power source (110) may also be configured to detach from medical device (100) and decouple from control module (120) or electrical connection (150). As a result, power source (110) may be completely separated from medical device (100) in some versions. As is readily apparent, this may allow the power source (110) to be removed to be recharged or reclaimed for resterilization and reuse, such as in accordance with various teachings herein. After recharging, or after an initial charge, power source (110) may be inserted or reinserted into medical device (100) and secured to housing (130) or internally within housing (130). Of course, medical device (100) may also allow power source (110) to be charged and/or recharged while power source (110) is still in or otherwise coupled relative to housing (130).

It should also be understood that control module (120) may be removed for servicing, testing, replacement, or any other purpose as will be apparent to one of ordinary skill in the art in view of the teachings herein. Further, end effector (140) may also be removable from medical device (100) for servicing, testing, replacement, or any other purpose as will be apparent to one of ordinary skill in the art in view of the teachings herein. While certain configurations of an exemplary medical device (100) have been described, various other ways in which medical device (100) may be configured will be apparent to those of ordinary skill in the art in view of the teachings herein.

By way of example only, medical devices (10, 100) and/or any other medical device referred to herein may be constructed in accordance with at least some of the teachings of U.S. Pat. No. 6,500,176; U.S. Pat. No. 6,783,524; U.S. Pat. No. 7,112,201; U.S. Pat. No. 7,125,409; U.S. Pat. No. 7,169,146; U.S. Pat. No. 7,186,253; U.S. Pat. No. 7,189,233; U.S. Pat. No. 7,220,951; U.S. Pat. No. 7,309,849; U.S. Pat. No. 7,311,709; U.S. Pat. No. 7,354,440; U.S. Pat. No. 7,381,209; U.S. Pat. No. 7,416,101; U.S. Pat. No. 7,738,971; U.S. Pub. No. 2006/0079874; U.S. Pub. No. 2007/0191713; U.S. Pub. No. 2007/0282333; U.S. Pub. No. 2008/0200940; U.S. Pub. No. 2009/0143797; U.S. Pub. No. 2009/0209990; U.S. Pub. No. 2010/0069940; U.S. Pub. No. 2011/0015660; U.S. Pat. Pub. No. 2011/0087218; U.S. Pat. App. No. 13/151,181 (published as US 2012-0210223 A1).

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### II. Exemplary Surgical Instrument with Multiple Transmission Assemblies

As noted above, transmission assembly (70, 200) may be provided as a disposable or otherwise replaceable assembly, thereby allowing surgical instrument (50) to be used many times by replacing transmission assembly (70, 200) between uses. In addition or in the alternative, it may be simply desirable to keep transmission assembly (70, 200) separate from surgical instrument (50) when surgical instrument (50) is not in use such that surgical instrument (50) and transmission assembly (70) may be stored separately. Furthermore, a user may wish to select from different transmission assemblies (70, 200) having different features, configurations, and/or operabilities depending on the particular context. For example, one transmission assembly (70, 200) may be equipped with a motorized stapler whereas another transmission assembly (70, 200) may be equipped with an RF end effector. In fact, several different transmission assemblies (70, 200) having vastly different functions may be used. It will be appreciated that it may be desirable to have a single handle assembly (60) capable of handling various different transmission assemblies (70, 200) such that multiple different handle assemblies (60) do not have to be used.

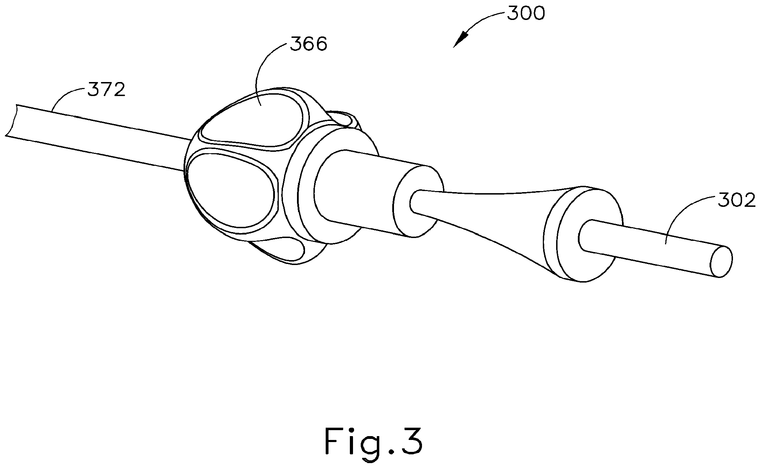

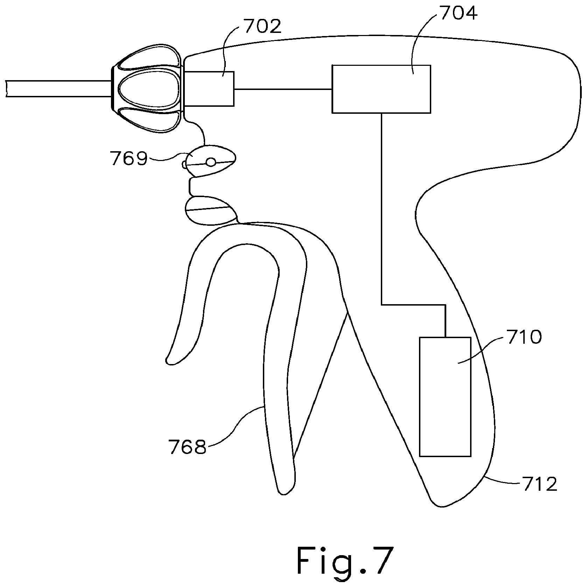

For example, FIGS. 3-6 show four different transmission assemblies (300, 400, 500, 600) for use with a handle assembly (700) as shown in FIG. 7. For example, a user may wish to use transmission assembly (300) with handle assembly (700) in one procedure, or for one part of a procedure. The user may then wish to use a different transmission assembly (400) with handle assembly (700). As will be described in further detail below, the user will be able to use both transmission assembly (300) and transmission assembly (400) with handle assembly (700) without requiring the user to change anything significant regarding handle assembly (700). In fact, handle assembly (700) may then also be used with any of transmission assemblies (500, 600) as will be apparent to one of ordinary skill in the art in view of the teachings herein. While the present example shows four different transmission assemblies (300, 400, 500, 600) it will be appreciated that any suitable number of transmission assemblies may be used as would be apparent to one of ordinary skill in the art in view of the teachings herein. It will further be appreciated that handle assembly (700) may function as a platform for the development of future transmission assemblies such that transmission assemblies may be developed with the understanding that they will be compatible with a common handle assembly (700).

As shown in FIG. 7, handle assembly (700) of the present example comprises a body (712), which houses a connector port (702). Connector port (702) is in communication with generator (704). Generator (704) may be integrated with a battery, or as shown in the exemplary version, generator (704) may be in electrical communication with battery (710). Battery (710) is operable to power generator (704). Handle assembly (700) further comprises toggle buttons (769) and trigger (768). Handle assembly (700) may have other exemplary configurations as would be apparent to one of ordinary skill in the art in view of the teachings herein.

FIG. 3 shows one merely exemplary transmission assembly (300) for use with handle assembly (700). Transmission assembly (300) comprises an ultrasonic transducer (302) connected to rotation knob (366). Rotation knob (366) connects to outer sheath (372) housing a waveguide operable to transfer ultrasonic vibrations through transmission assembly (300) to an end effector (not shown) located at a distal end of transmission assembly (300). Transducer (302) is operable to couple with connector port (702) in any suitable way as would be apparent to one of ordinary skill in the art in view of the teachings herein. For example, transducer (302) may be structured so as to screw into connector port (702). In other exemplary versions, transducer (302) may be operable to snap into connector port (702). In yet other merely exemplary versions, transducer (302) may be connected to connector port (702) and secured using latches to secure transducer (302). Once transmission assembly (300) is connected to handle assembly (700), it will be appreciated that actuation of toggle buttons (769) and/or trigger (768) is operable to activate generator (704). Generator (704) communicates power to connector port (702), which communicates power to transducer (302) as a result of being in communication with transducer (302). Transducer (302) then converts the power into ultrasonic energy, which may then be delivered to a surgical site via the end effector at the distal end of transmission assembly (300). Furthermore, it will be appreciated that toggle buttons (769) and/or trigger (768) may be operable to control the end effector at the distal end of transmission assembly (300). Other suitable configurations may be used as would be apparent to one of ordinary skill in the art in view of the teachings herein.

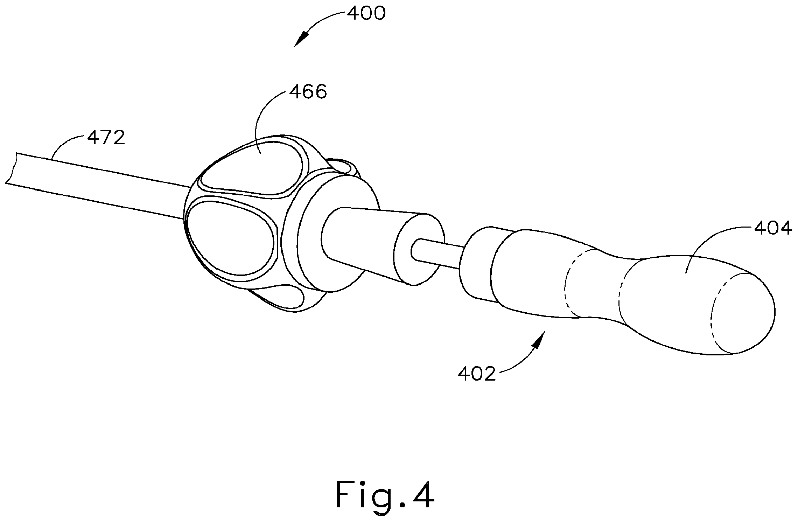

FIG. 4 shows an exemplary version of a transmission assembly (400) for use with handle assembly (700). Transmission assembly (400) comprises an ultrasonic transducer (402) connected to rotation knob (466). Rotation knob (466) connects to outer sheath (472) housing a waveguide operable to transfer ultrasonic vibrations through transmission assembly (400) to an end effector. Transducer (402) in the present example is contained within a contoured housing (404). It will be appreciated that contoured housing (404) may have a shape complementary to connector port (702). The present example shows contoured housing (404) having an elongated, rounded shape. However, any suitable shape may be used as would be apparent to one of ordinary skill in the art in view of the teachings herein. It will be appreciated that given the elongated, rounded shape of housing (404) of the present example, a user may easily grip housing (404) prior to coupling housing (404) with connector port (702). It will further be appreciated that housing (404) may be constructed of an electrically conductive material such that when transducer (402) is coupled with connector port (702), generator (704) may be operable to deliver energy to transducer (402) through housing (404). It will be appreciated that transmission assembly (400) may otherwise be configured and operable similar to transmission assembly (300) as described above.

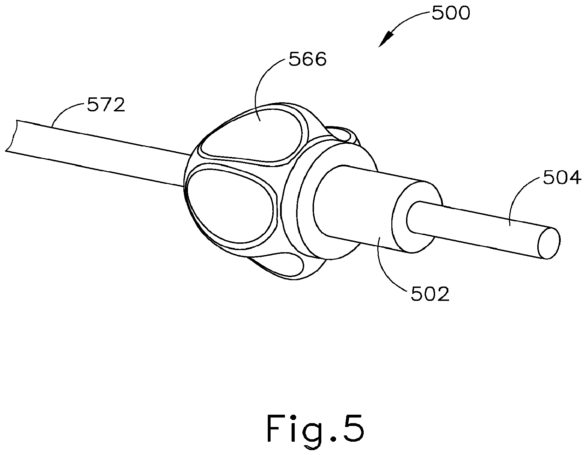

FIG. 5 shows yet another exemplary version of a transmission assembly (500) for use with handle assembly (700). Transmission assembly (500) comprises an RF module (502) connected to rotation knob (566). Rotation knob (566) connects to outer sheath (572) housing a wire or other conduit operable to conduct RF power through transmission assembly (500). In the exemplary version, module (502) is connected to a connector rod (504) operable to engage connector port (702), which thereby electrically couples module (502) to generator (704). Therefore, generator (704) may deliver energy to module (502). Module (502) then is operable to convert electrical energy to RF frequencies that may be delivered to a surgical site via an end effector at a distal end of transmission assembly (500). Connector rod (504) has a shape complementary to connector port (702). While the exemplary version depicts module (502) and connector rod (504) as separate components connected to each other, it will be appreciated that module (502) and connector rod (504) may be constructed as a single integrated component. In some versions, module (502) may comprise a positive contact whereas connector rod (504) may comprise a negative contact or vice versa. Other suitable configurations will be apparent to one of ordinary skill in the art in view of the teachings herein.

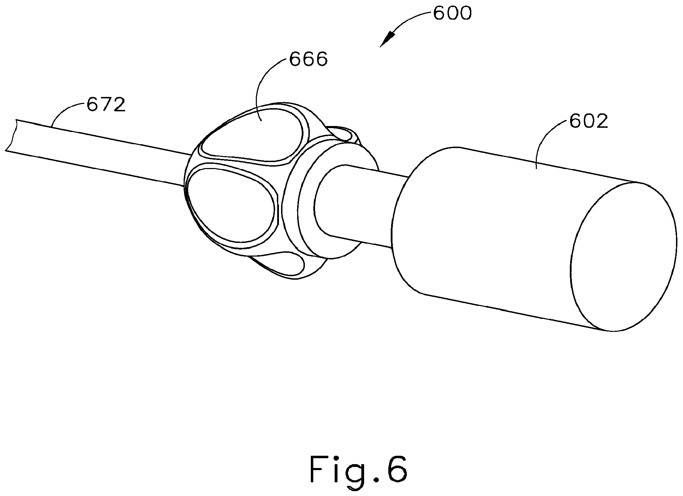

FIG. 6 depicts an exemplary version of a transmission assembly (600) for use with handle assembly (700). Transmission assembly (600) of the exemplary version is operable to apply one or more surgical staples to a surgical site. Transmission assembly (600) comprises a motor (602) in communication with generator (704) such that generator (704) is operable to power motor (602) once transmission assembly (600) is connected to connector port (702). Once powered, motor (602) may be used in communication with an end effector configured to apply staples to a surgical site where motor (602) is operable to help drive such staples. For example, transmission assembly (600) may be used in accordance with the teachings of U.S. Patent No. 7,416,101, entitled "Motor-driven Surgical Cutting and Fastening Instrument with Loading Force Feedback," issued August 26, 2008

As can be seen in the above exemplary versions, transmission assemblies (300, 400, 500, 600) are operable to work in different modalities where each of transmission assemblies (300, 400, 500, 600) may require different instructions for proper operation. Generator (704) may be integrated with a programmable chip such that generator (704) may store information regarding the operation of transmission assemblies (300, 400, 500, 600). Each transmission assembly (300, 400, 500, 600) may comprise, for example, an identifier such that when transmission assembly (300, 400, 500, 600) and connector port (702) establish communication, generator (704) may be able to determine the type of transmission assembly (300, 400, 500, 600) attached. Thereafter, generator (704) can use instructions stored in the programmable chip to properly operate transmission assembly (300, 400, 500, 600) according to the specific modality of transmission assembly (300, 400, 500, 600) attached.

In yet other exemplary versions, connector port (702) may be operable to detect the identity of transmission assembly (300, 400, 500, 600) attached without transmission assembly (300, 400, 500, 600) having to directly identify itself to connector port (702). For example, connector port (704) may be configured to detect the impedance, number of connection contacts, etc., or any other suitable feature to determine which transmission assembly (300, 400, 500, 600) is connected to connector port (702).

It will be understood that in some exemplary versions, generator (704) and other components of handle assembly (700) do not necessarily need to contain any sort of processor or computing component. Such components may, in some versions, reside entirely in transmission assembly (300, 400, 500, 600). As a result, it will be appreciated that updated firmware may be stored and shipped with transmission assembly (300, 400, 500, 600), thereby allowing transmission assembly (300, 400, 500, 600) to have the latest version of any software for use with handle assembly (700). In some versions, generator (704) simply provides power to transmission assembly (300, 400, 500, 600) and/or merely follows instructions from transmission assembly (300, 400, 500, 600).

In some exemplary versions, it will be appreciated that the only connections between connectors may consist of electrical connections for providing power to an end effector of transmission assembly (300, 400, 500, 600) as well as a force transfer mechanism operable to engage and control any mechanically moving parts of transmission assembly (300, 400, 500, 600). It will be appreciated that such a configuration will allow for a simpler construction of transmission assembly (300, 400, 500, 600), which may be shipped separately from handle assembly (700). In yet other exemplary versions, it will be appreciated that any suitable portion of the electronics may be controlled from transmission assembly (300, 400, 500, 600) wherein another suitable portion of the electronics may be controlled from handle assembly (700). As a result, the number of electronic components may be divided between handle assembly (700) and transmission assembly (300, 400, 500, 600) in any suitable manner as would be apparent to one of ordinary skill in the art in view of the teachings herein.

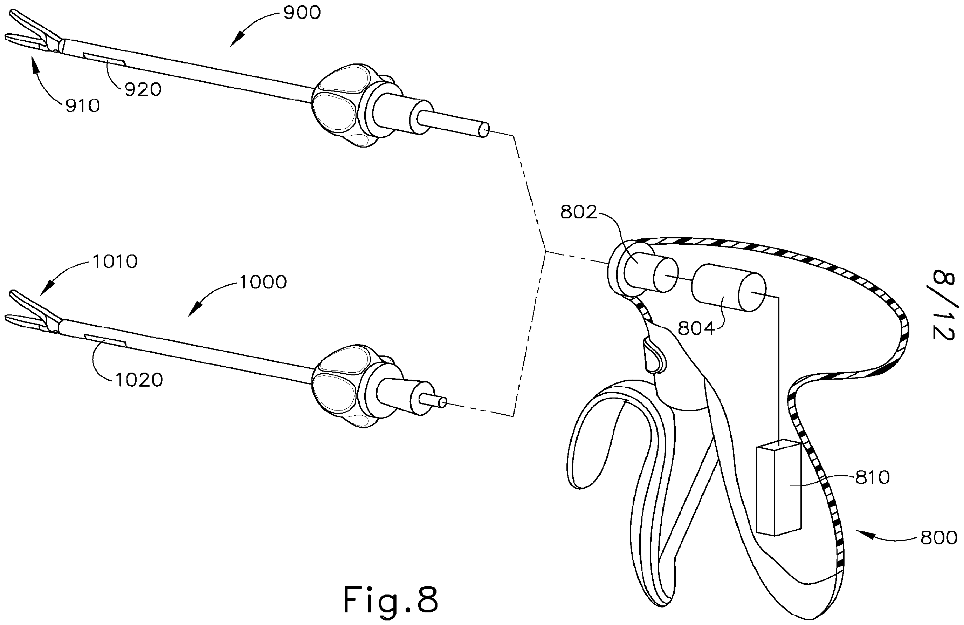

FIG. 8 shows an exemplary handle assembly (800) according to an embodiment of the present invention, with two transmission assemblies (900, 1000), which are operable to be in communication with handle assembly (800). Transmission assembly (900) is operable as an RF transmission assembly and comprises an end effector (910). End effector (910) comprises a memory module (920). Memory module (920) may comprise an integrated memory chip, a removeable memory chip, or any other suitable memory device as would be apparent to one of ordinary skill in the art in view of the teachings herein. RF transmission assembly (900) is operable to be connected to connector port (802) of handle assembly (800), which also comprises processor (804) and power source (810). Memory module (920) comprises a set of instructions for operation of handle assembly (800) in the RF modality. Once connected to connector port (802), memory module (920) transmits these instructions to processor (804) regarding the operation of handle assembly (800). In other words, memory module (920) programs processor (804), and processor (804) thereafter executes the program through RF transmission assembly (900). After delivering instructions to handle assembly (800), handle assembly (800) becomes operable to operate in the appropriate modality of RF transmission assembly (900). In the exemplary version, RF transmission assembly (900) is operable to deliver RF energy to a surgical site. It will be appreciated that memory module (920) may be upgraded with firmware separately from handle assembly (800).

Also depicted is an ultrasonic transmission assembly (1010), which is operable to deliver ultrasonic signal to a surgical site. Ultrasonic transmission assembly (1010) comprises a memory module (1020) and end effector (1010). Memory module (1020) contains instructions for operating transmission assembly (1010). Thus, as with RF transmission assembly (900) as discussed above, it will be appreciated that once ultrasonic transmission assembly (1000) is connected to connector port (802), memory module (1020) transfers instructions to processor (804) of handle assembly (800) operable to operate ultrasonic transmission assembly (1000) in the ultrasonic modality. As a result, it will be appreciated that a single handle assembly (800) may be operable for use with both ultrasonic transmission assembly (1000) and RF transmission assembly (900).

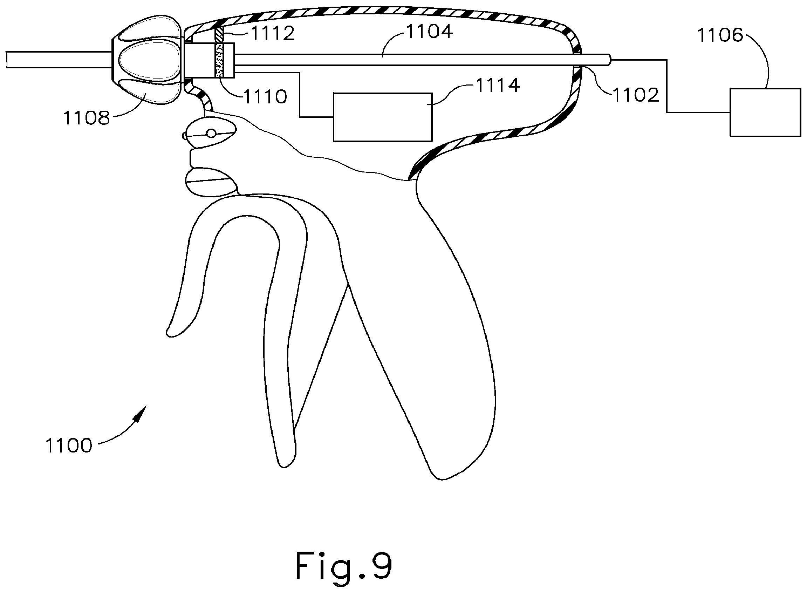

FIG. 9 shows an exemplary handle assembly (1100) operable for use with bipolar and monopolar RF electrosurgical end effectors (80). Handle assembly (1100) comprises a monopolar opening (1102) operable to receive a positive electrode (1104) long enough to extend through handle assembly (1100). In some versions, positive electrode (1104) may be selectively covered with a cap or other suitable device (e.g., during operation in a bipolar RF mode, etc.). Handle assembly (1100) is operable to receive a transmission assembly (1108). Transmission assembly (1108) comprises a negative contact (1110) operable to contact with a negative post (1112) contained in handle assembly (1100). Positive electrode (1104) is operable to contact an external module (1106) where external module (1106) comprises either a grounding pad or a generator output, which might include an RF generator. In the event that external module (1106) comprises a generator output, it will be appreciated that connecting positive electrode (1104) to the generator output is operable to disconnect positive electrode (1104) from other electronic components contained within handle assembly (1100). For example, a generator (1114) contained within handle assembly (1100) may be in selective communication with transmission assembly (1108) and be operable to detect if transmission assembly (1108) comprises a monopolar transmission assembly (1108). If so, then generator (1114) may simply no longer deliver power to transmission assembly (1108). In the scenario where generator (1114) detects that transmission assembly (1108) comprises a bipolar transmission assembly (1108), generator (1114) may then be operable to deliver power to transmission assembly (1108) for use. In the event that external module (1106) comprises a grounding pad, grounding pad may be placed in contact with a patient. As a result, in a bipolar setting, a secondary wire/contact of transmission assembly (1108) in communication with negative contact (1110) is in communication with negative post (1112). In a monopolar setting, transmission assembly (1108) is not in communication with negative post (1112) such that a grounding pad serves as the only ground return in communication with positive electrode (1104).

In some versions, external module (1106) may comprise an electrosurgical generator operable to supply power in the monopolar setting such that the grounding pad would be in communication with the electrosurgical generator. Furthermore in a monopolar setting, the disconnection of transmission assembly (1108) with negative post (1112) may occur mechanically and/or electrically. For example, in a mechanical disconnection, connecting a monopolar electrode may push transmission assembly (1108) slightly forward, causing negative post (1112) to disengage from negative contact (1110). In an electrical disconnection, the polarity of transmission assembly (1108) may be detected using a sensor, RFID, chip, or other means, and circuit between negative post (1112) and negative contact (1110) may be opened in response to detection that a monopolar generator is activated. In some such versions, a switch may be used to perform the disconnection. Other suitable ways in which negative post (1112) may be mechanically and/or electrically decoupled from negative contact (1110) and/or other components will be apparent to those of ordinary skill in the art in view of the teachings herein.

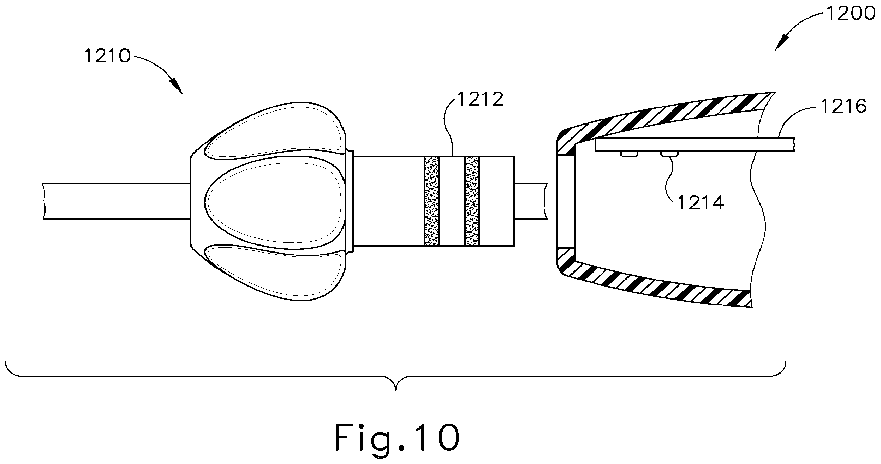

It will be appreciated that in any of the above-described transmission assemblies and handle assemblies, it may be desirable to determine the identity of the transmission assembly for: identification of the modality, the polarity, and/or other characteristics associated with the transmission assembly. FIG. 10 depicts an exemplary handle assembly (1200) having an identification device (1212) connected to transmission assembly (1210). In the exemplary version, when transmission assembly (1210) is coupled with handle assembly (1200), identification device (1212) is operable to establish electromechanical communication with contacts (1214), which are in communication with a controller board (1216). Once contacts (1214) engage controller board (1216), it will be appreciated that controller board (1216) may perform, for example, an electronic handshake or other suitable protocol to determine the identity of transmission assembly (1210), which may include relevant information regarding transmission assembly's (1210) modality, polarity, etc. Other suitable information may be gathered as well such as the serial number, firmware version, etc. of transmission assembly (1210). In the exemplary version, identification device (1212) may comprise conductive rings, grooves, and/or protruding rings for establishing contact with controller board (1216) and identifying transmission assembly (1210), but any suitable connection feature may be used as would be apparent to one of ordinary skill in the art in view of the teachings herein. The conductive rings, grooves, and/or protruding rings are operable to close certain portions of the circuit of controller board (1216) based on what particular portions of contacts (1214) engage the physical features of conductive rings, grooves, and/or protruding rings. Based on what portions of controller board (1216) are closed, controller board (1216) may experience and be operable to monitor changes in current, voltage, etc. As a result of these changes in current, voltage, etc., controller board (1216) is operable to determine the identity and characteristics of transmission assembly (1210) including the identity, modality, etc. of an end effector (not shown) attached to the distal end of transmission assembly (1210).

In some versions, identification device (1212) may comprise one or more interrupted rings such that a revolution of transmission assembly (1210) within handle assembly (1200) may result in a discrete number of contacts with contacts (1214) of controller board (1216) in a particular pattern indicative of the modality, etc. of transmission assembly (1210). As a result, controller board (1216) may also be operable to deduce the identity of transmission assembly (1210) by the number of interruptions caused by transmission assembly (1210) during a revolution of transmission assembly (1210). For example, if during the course of one revolution, identification device (1212) engages contacts (1214) four times, then it may be indicative of transmission assembly (1210) operating in the RF modality. In some versions, identification device (1212) may include conductive rings, grooves, reed switches, magnets, and/or protruding rings that are different based on the modality of transmission assembly (1210) such that when contacts (1214) engage identification device (1212), contacts (1214) communicate to controller board (1216) information regarding the identity and/or modality of transmission assembly (1210). In addition to the conductive rings, grooves, reed switches, magnets, and/or protruding rings merely touching contacts (1214), it will be appreciated that in some versions, conductive rings, grooves, reed switches, magnets, and/or protruding rings may be operable to engage switches analogous to how a key engages tumblers in a lock, thereby closing circuits in controller board (1216), which can be used to determine the identity of transmission assembly (1210). In the present example, the conductive rings, grooves, reed switches, magnets, and/or protruding rings are located on the side of identification device (1212), but it will be appreciated that conductive rings, grooves, reed switches, magnets, and/or protruding rings may be located on the proximal face of identification device (1212) as well.

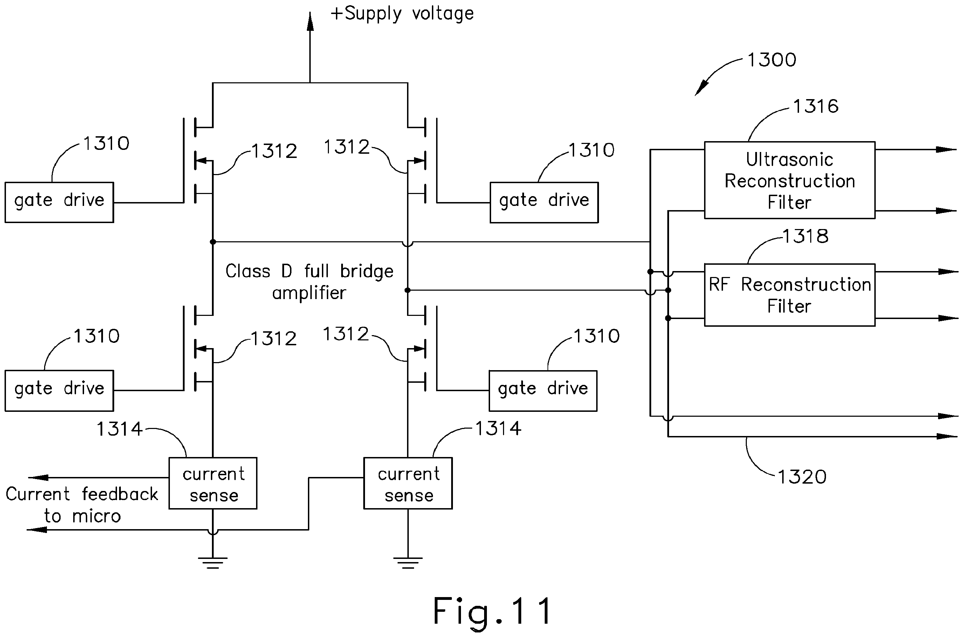

FIG. 11 shows an exemplary amplifier (1300) according to an embodiment of the present invention, operable to be used with any of the transmission assemblies discussed above. It will be appreciated that depending on the type of transmission assembly, different power and/or frequency needs may be required. For instance, an ultrasonic transmission assembly may require a frequency of 20-100 kHz, whereas an RF electrosurgical transmission assembly may require a frequency of 200-500 kHz, whereas a motor-driven stapling device may require DC power. Amplifier (1300) is operable to be used to power any suitable transmission assembly as would be apparent to one of ordinary skill in the art in view of the teachings herein including, but not limited to: transmission assemblies operable to deliver harmonic energy, RF energy, thermal energy (through a heated electrode or end effector) or staples to a surgical site. Amplifier (1300) comprises a class D full bridge amplifier having a plurality of MOSFETs (1312) in communication with a plurality of gate drives (1310) and a plurality of current sensors (1314) operable to monitor current. Amplifier (1300) further comprises an ultrasonic reconstruction filter (1316) and an RF reconstruction filter (1318). Ultrasonic reconstruction filter (1316) is operable to deliver power to a transmission assembly configured for delivering ultrasonic energy to a surgical site. RF reconstruction filter (1318) is operable to deliver power to a transmission assembly configured to deliver RF energy to a surgical site. Finally, a direct line (1320) may be used to transmit energy to a motor which may be used, for example to drive surgical staples into a surgical site. While the exemplary amplifier (1300) utilizes a plurality of MOSFETs (1312) for amplification, it will be appreciated that any suitable transistor may be used as would be apparent to one of ordinary skill in the art in view of the teachings herein. Furthermore, it will be understood that amplifier (1300) may operate the above-noted transmission assemblies without requiring switching relays and without requiring an output transformer. While the exemplary version utilizes a Class D full bridge amplifier, it will be appreciated that other suitable amplifier topologies may be used. For example, a full bridge amplifier, a half bridge amplifier, a single ended amplifier, or any other suitable amplifier may be used. Furthermore, in addition or in the alternative to a Class D amplifier, a Class E, Class F, Class G, Class H, Doherty, Class T, Class Z, or any other suitable amplifier may be used.

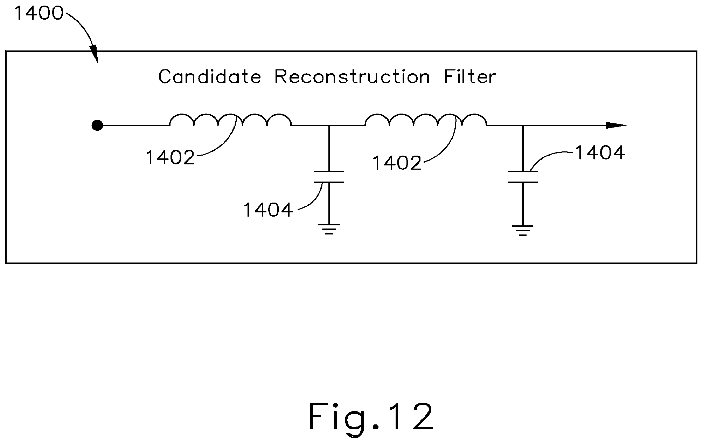

FIG. 12 depicts an exemplary reconstruction filter (1400) comprising a pair of inductors (1402) and a pair of capacitors (1404), a 'Pi-L' filter. Reconstruction filter (1400) is operable to be used in conjunction with or as ultrasonic reconstruction filter (1316) and/or RF reconstruction filter (1318). In some versions, reconstruction filter (1400) is operable for use with any suitable class D amplifier. Reconstruction filter (1400), in conjunction with an amplifier, utilizes a variation of pulse width modulation to generate a sinusoidal output for use by a transmission assembly where the output signal has an overall low harmonic distortion. It will be appreciated that the phase of the pulse width modulation may be controlled and/or modified thereby controlling the phase of the sinusoidal output of reconstruction filter (1400).

It will also be appreciated that reconstruction filter (1400) is operable to allow control of the phase of output signal of amplifier (1300) to be maintained such that the output signal remains agile/flexible without undue delay between any instructions regarding changing phase in relation to when any phase change may eventually take effect. For example, reconstruction filter (1400) may utilize variable capacitors and/or variable inductors or other means to change the circuit elements such that the user may control the phase output of filter (1400) as well as, or in the alternative, control the width of filter (1400). It will further be appreciated that the pulse width modulation supplied to filter (1400) is sufficiently high such that the phase of the output of filter (1400) may be modified without compromising the sinusoidal output of filter (1400). Furthermore, it will also be appreciated that the timing of the pulse width modulation need not be uniform. In some versions, the pulse width modulation may be sinusoidally spaced in such a way so as to control the output amplitude of filter (1400).

It will be appreciated that amplifier (1300) enables the use of batteries to drive ultrasonic and/or RF end effectors without requiring unduly large electrical components that might otherwise be required for handling the delivery of power to an ultrasonic device, an RF based device, and/or a DC utilizing device. In other exemplary versions, any suitable power source (AC or DC) may be used as an input for amplifier. It will also be appreciated that during the operation of amplifier (1300), transistors contained in amplifier (1300) remain continuously or nearly continuously in saturation such that very little voltage is dropped across any connected devices when devices are connected to or removed from communication with amplifier (1300).

In some versions, a feedback loop may be used to produce a more desirable output of amplifier (1300). In particular, a feedback system may be in communication with amplifier (1300) for determining what may be required to compensate for non-linearity in the output of amplifier (1300). Accordingly, the drive waveform input to amplifier (1300) may be selectively pre-distorted in such a way that is operable to eliminate undesirable behavior in amplifier (1300) as determined by the feedback system. Furthermore, the drive waveform input may also be selectively pre-distorted to compensate for known or unknown non-linearity in the output of amplifier (1300). In some versions, the output voltage of amplifier (1300) may be monitored and analyzed in between applications of power, thereby allowing the user to determine the state of the electro-mechanical system associated with amplifier (1300) or the surgical instrument in communication with amplifier (1300). As a result of analyzing the voltage response, the input signal for amplifier (1300) may be pre-distorted or modified to compensate for or eliminate undesirable voltage behavior.

### III. Miscellaneous

It is contemplated that various teachings herein may be combined in numerous ways, and it should be understood that none of the teachings herein are intended to represent the limits of the inventors' contemplation. Various other examples of how several features of the surgical instruments described herein may be carried out in practice will be apparent to those of ordinary skill in the art in view of the teachings herein, and those examples are well within the inventors' contemplation.

Versions of the devices disclosed herein can be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, versions of the device may be disassembled, and any number of the particular pieces or parts of the device may be selectively replaced or removed in any combination. Upon cleaning and/or replacement of particular parts, versions of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a surgical facility. A device may also be sterilized using any other technique known in the art, including but not limited to beta or gamma radiation, ethylene oxide, or steam.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical apparatus comprising:
(a) a handle assembly (800);
(b) a first transmission assembly (900) for selective mechanical and electrical communication with a connector port (802) of the handle assembly (800), wherein the first transmission assembly (900) includes an end effector and is operable to deliver energy to a surgical site;
(c) a second transmission assembly (1000) for selective mechanical and electrical communication with the connector port (802) of the handle assembly (800), wherein the second transmission assembly (1000) includes an end effector and is operable to deliver energy to a surgical site, wherein the first transmission assembly (900) and the second transmission assembly (1000) are configured to operate in different modalities;
(d) an amplifier (1300) in communication with the handle assembly (800); and wherein:
(e) at least two electric reconstruction filters communication with the amplifier and the connector port (1312), wherein the at least two reconstruction filters comprise an ultrasonic reconstruction filter (1316) operable to deliver power to a transmission assembly configured for delivering ultrasonic energy to a surgical site and an RF reconstruction filter (1318) operable to deliver power to a transmission assembly configured to deliver RF energy to a surgical site. (1318).

2. The apparatus of claim 1, wherein the first transmission assembly (900) is configured to deliver ultrasonic energy, wherein the second transmission assembly (800) is configured to deliver RF energy.

3. The apparatus of claim 1, wherein the first transmission assembly (900) comprises an identification device.

4. The apparatus of claim 3, wherein the identification device comprises at least one interrupted contact (1212)and the handle assembly (800) comprises a controller board (1216) operable to communicate with the first transmission assembly, such that a revolution of the first transmission assembly (900) within the handle assembly (800) results in a discrete number of contacts between the at least one interrupted contact (1212) and contacts (1214) of the controller board (1216) in a particular pattern indicative of the modality of the first transmission assembly (900).

5. The apparatus of claim 1, wherein the handle assembly (800) is configured to receive bipolar and monopolar RF transmission assemblies.

6. The apparatus of claim 1, wherein the first transmission assembly (900) comprises a memory module.

7. The apparatus of claim 6, wherein the memory module is configured to receive updatable firmware.

8. The apparatus of claim 6, wherein the memory module is configured to transmit instructions to the handle assembly (800).

9. The apparatus of claim 1, further comprising a third transmission assembly (600) in selective communication with the handle assembly (800), wherein the third transmission assembly (600) is configured to deliver a plurality of staples to a surgical site.

10. The apparatus of claim 1, wherein the handle assembly (800) comprises a generator (704) having a programmable chip operable to store instructions regarding operating the handle assembly (800) with different modalities.

11. The apparatus of claim 1, wherein the handle assembly (800) comprises an opening (1102) operable to receive an electrode (1104) of a monopolar transmission assembly.

12. The apparatus of claim 1, wherein at least one reconstruction filter is operable to provide pulse width modulation.

13. The apparatus of claim 1, wherein at least one reconstruction filter (1400) comprises a pair of inductors (1402) and a pair of capacitors (1404) operable to be used as the ultrasonic reconstruction filter (1316) and the RF reconstruction filter (1318).

## Patentansprüche

1. Chirurgischer Apparat, umfassend:
(a) eine Griffbaugruppe (800);
(b) eine erste Übertragungsbaugruppe (900) zur selektiven mechanischen und elektrischen Kommunikation mit einem Konnektoranschluss (802) der Griffbaugruppe (800), wobei die erste Übertragungsbaugruppe (900) einen Endeffektor einschließt und funktional ist, um Energie an einen Operationssitus abzugeben;
(c) eine zweite Übertragungsbaugruppe (1000) zur selektiven mechanischen und elektrischen Kommunikation mit dem Konnektoranschluss (802) der Griffbaugruppe (800), wobei die zweite Übertragungsbaugruppe (1000) einen Endeffektor einschließt und funktional ist, um Energie an einen Operationssitus abzugeben, wobei die erste Übertragungsbaugruppe (900) und die zweite Übertragungsbaugruppe (1000) ausgestaltet sind, um in unterschiedlichen Modalitäten zu arbeiten;
(d) einen Verstärker (1300) in Kommunikation mit der Griffbaugruppe (800); und wobei:
(e) mindestens zwei elektrische Rekonstruktionsfilter in Kommunikation mit dem Verstärker und dem Konnektoranschluss (1312) sind, wobei die mindestens zwei Rekonstruktionsfilter ein Ultraschallrekonstruktionsfilter (1316), das funktional ist, um Leistung an eine Übertragungsbaugruppe abzugeben, die zum Abgeben von Ultraschallenergie an einen Operationssitus ausgestaltet ist, und ein HF-Rekonstruktionsfilter (1318) umfassen, das funktional ist, um Leistung an eine Übertragungsbaugruppe abzugeben, die ausgestaltet ist, um HF-Energie an einen Operationssitus abzugeben.

2. Apparat nach Anspruch 1, wobei die erste Übertragungsbaugruppe (900) ausgestaltet ist, um Ultraschallenergie abzugeben, wobei die zweite Übertragungsbaugruppe (800) ausgestaltet ist, um HF-Energie abzugeben.

3. Apparat nach Anspruch 1, wobei die erste Übertragungsbaugruppe (900) eine Identifikationsvorrichtung umfasst.

4. Apparat nach Anspruch 3, wobei die Identifikationsvorrichtung mindestens einen unterbrochenen Kontakt (1212) umfasst, und die Griffbaugruppe (800) eine Steuerplatine (1216) umfasst, die funktional ist, um mit der ersten Übertragungsbaugruppe zu kommunizieren, so dass eine Umdrehung der ersten Übertragungsbaugruppe (900) innerhalb der Griffbaugruppe (800) zu einer diskreten Anzahl an Kontakten zwischen dem mindestens einen unterbrochenen Kontakt (1212) und Kontakten (1214) der Steuerplatine (1216) in einem bestimmten Muster führt, welches die Modalität der ersten Übertragungsbaugruppe (900) angibt.

5. Apparat nach Anspruch 1, wobei die Griffbaugruppe (800) ausgestaltet ist, um bipolare und monopolare HF-Übertragungsbaugruppen aufzunehmen.

6. Apparat nach Anspruch 1, wobei die erste Übertragungsbaugruppe (900) ein Speichermodul umfasst.

7. Apparat nach Anspruch 6, wobei das Speichermodul ausgestaltet ist, um updatefähige Firmware aufzunehmen.

8. Apparat nach Anspruch 6, wobei das Speichermodul ausgestaltet ist, um Anweisungen an die Griffbaugruppe (800) zu übertragen.

9. Apparat nach Anspruch 1, des Weiteren umfassend eine dritte Übertragungsbaugruppe (600) in selektiver Kommunikation mit der Griffbaugruppe (800), wobei die dritte Übertragungsbaugruppe (600) ausgestaltet ist, um eine Vielzahl von Klammern an einen Operationssitus abzugeben.

10. Apparat nach Anspruch 1, wobei die Griffbaugruppe (800) einen Generator (704) mit einem programmierbaren Chip umfasst, der funktional ist, um Anweisungen zum Arbeiten mit der Griffbaugruppe (800) mit unterschiedlichen Modalitäten zu speichern.

11. Apparat nach Anspruch 1, wobei die Griffbaugruppe (800) eine Öffnung (1102) umfasst, die funktional ist, um eine Elektrode (1104) einer monopolaren Übertragungsbaugruppe aufzunehmen.

12. Apparat nach Anspruch 1, wobei das mindestens eine Rekonstruktionsfilter funktional ist, um Pulsbreitenmodulation bereitzustellen.

13. Apparat nach Anspruch 1, wobei mindestens ein Rekonstruktionsfilter (1400) ein Paar Spulen (1402) und ein Paar Kondensatoren (1404) umfasst, die funktional sind, um als das Ultraschallrekonstruktionsfilter (1316) und das HF-Rekonstruktionsfilter (1318) verwendet zu werden.

## Revendications

1. Appareil chirurgical comportant :
(a) un ensemble (800) de poignée ;
(b) un premier ensemble (900) de transmission destiné à une communication mécanique et électrique sélective avec un port (802) de connecteur de l'ensemble (800) de poignée, le premier ensemble (900) de transmission comprenant un effecteur terminal et étant exploitable pour délivrer de l'énergie à un site chirurgical ;
(c) un deuxième ensemble (1000) de transmission destiné à une communication mécanique et électrique sélective avec le port (802) de connecteur de l'ensemble (800) de poignée, le deuxième ensemble (1000) de transmission comprenant un effecteur terminal et étant exploitable pour délivrer de l'énergie à un site chirurgical, le premier ensemble (900) de transmission et le deuxième ensemble (1000) de transmission étant configurés pour fonctionner dans des modalités différentes ;
(d) un amplificateur (1300) en communication avec l'ensemble (800) de poignée ; et :
(e) au moins deux filtres électriques de reconstitution en communication avec l'amplificateur et le port (1312) de connecteur, lesdits au moins deux filtres de reconstitution comportant un filtre (1316) de reconstitution ultrasonique exploitable pour délivrer une puissance à un ensemble de transmission configuré pour délivrer de l'énergie ultrasonique à un site chirurgical et un filtre (1318) de reconstitution à RF exploitable pour délivrer une puissance à un ensemble de transmission configuré pour délivrer de l'énergie RF à un site chirurgical.

2. Appareil selon la revendication 1, le premier ensemble (900) de transmission étant configuré pour délivrer de l'énergie ultrasonique, le deuxième ensemble (800) de transmission étant configuré pour délivrer de l'énergie RF.

3. Appareil selon la revendication 1, le premier ensemble (900) de transmission comportant un dispositif d'identification.

4. Appareil selon la revendication 3, le dispositif d'identification comportant au moins un contact interrompu (1212) et l'ensemble (800) de poignée comportant une carte (1216) de commande exploitable pour communiquer avec le premier ensemble de transmission, de telle façon qu'un tour du premier ensemble (900) de transmission à l'intérieur de l'ensemble (800) de poignée se traduise par un nombre discret de contacts entre le ou les contacts interrompus (1212) et des contacts (1214) de la carte (1216) de commande selon un motif particulier indicatif de la modalité du premier ensemble (900) de transmission.

5. Appareil selon la revendication 1, l'ensemble (800) de poignée étant configuré pour recevoir des ensembles de transmission RF bipolaires et unipolaires.

6. Appareil selon la revendication 1, le premier ensemble (900) de transmission comportant un module de mémoire.

7. Appareil selon la revendication 6, le module de mémoire étant configuré pour recevoir un microprogramme susceptible d'être mis à jour.

8. Appareil selon la revendication 6, le module de mémoire étant configuré pour transmettre des instructions à l'ensemble (800) de poignée.

9. Appareil selon la revendication 1, comportant en outre un troisième ensemble (600) de transmission en communication sélective avec l'ensemble (800) de poignée, le troisième ensemble (600) de transmission étant configuré pour délivrer une pluralité d'agrafes à un site chirurgical.

10. Appareil selon la revendication 1, l'ensemble (800) de poignée comportant un générateur (704) doté d'une puce programmable exploitable pour stocker des instructions concernant l'exploitation de l'ensemble (800) de poignée avec différentes modalités.

11. Appareil selon la revendication 1, l'ensemble (800) de poignée comportant une ouverture (1102) exploitable pour recevoir une électrode (1104) d'un ensemble de transmission unipolaire.

12. Appareil selon la revendication 1, au moins un filtre de reconstitution étant exploitable pour assurer une modulation de largeur d'impulsions.

13. Appareil selon la revendication 1, au moins un filtre (1400) de reconstitution comportant une paire de inductances (1402) et une paire de condensateurs (1404) exploitables pour être utilisés en tant que filtre (1316) de reconstitution ultrasonique et que filtre (1318) de reconstitution à RF.
